# EUROPEAN PATENT APPLICATION

(11) **EP 2 019 096 A1**
(43) Date of publication of application: **28.01.2009**
(21) Application number: 07113218.7
(22) Date of filing: 26.07.2007
(51) Int. Cl.: C07D 295/088

(54) **Process for obtaining Cetirizine dihydrochloride**

(71) Applicant: Cosma S.p.A., 24040 Ciserano (IT)
(72) Inventor: Lussana, Massimiliano, 24020, GORLE (IT); Vigano', Enrico, 22040, LURAGO D'ERBA (IT); Pizzatti, Enrica, 23020, POGGIRIDENTI (IT)
(74) Representative: Gervasi, Gemma

(57) **Abstract**

Process for the synthesis of cetirizine dihydrochloride, wherein
(a) a solution of {2-[4-(α-phenyl-p-chlorobenzyl)piperazin-1-yl]}ethanol in 1-7 volumes, referred to the weight of {2-[4-(α-phenyl-p-chlorobenzyl)piperazin-1-yl]}ethanol, of an organic solvent having a boiling point higher than 90°C and being chosen from the group consisting of aliphatic, cycloalifatic or aromatic solvents is provided, whereafter
(b) per equivalent of {2-[4-(α-phenyl-p-chlorobenzyl)piperazin-1-yl]}ethanol employed, 1-2 equivalents of a metal haloacetate or of haloacetic acid, as well as 3-7 equivalents of an alkaly metal hydroxyde are added to the solution as per (a), providing a reaction mixture, where 0,05-0,3 volumes, referred to the weight of {2-[4-(α-phenyl-p-chlorobenzyl)piperazin-1-yl]}ethanol employed, of water and 0,1-1,2 volumes, referred to the weight of {2-(4-(α-phenyl-p-chlorobenzyl)piperazin-1-yl]}ethanol employed, of a polar aprotic, water miscible solvent are added, keeping the internal temperature of the reaction mixture below 60°C, whereafter
(c) the cetirizine base formed within the reaction mixture is converted into its dihydrochloride salt and isolated as such.

## Description

### Field of the invention.

The present invention relates to a new, improved synthetical process for obtaining cetirizine (C21H25ClN2O3, CAS 83881-51-0, MW 388.89), in particular its dihydrochloride salt cetirizine•2Hl (MW 461.80). The new process herein described features eased industrial applicability, is safe and displays improved yields and improved purity of the product obtained.

### Technical Background.

Cetirizine, and more in particular its dihydrochloride, that is to say the twofold hydrochloric acid addition salt of (R,S)-[2-[4-[(4-chlorophenyl)phenylmethyl]piperazin-1-yl]ethoxy]acetic acid (C21H25ClN2O3•2HCl) is a pharmaceutical indicated for the treatment of allergic syntoms and one of its major advantages is the absence of collateral effects on the central nervous system.
Cetirizine dihydrochloride is a molecule of great success on the market, which went recently off Patent, and hence different methods of its synthesis have been studied.

According to the "basic" Patent EP 58 146, an ester or an amide of cetirizine are hydrolized with aqueous potassium hydroxide, to the cetirizine potassium salt to be converted in its turn into cetirizine and its respective hydrochloride and dihydrochloride. The yield of this process is low.

GB 2 225 320 proposed later a synthetic strategy according to which {2-[4-(α-phenyl-p-chlorobenzyl)piperazin-1-yl]}ethanol (hereinafter "ethanolic precursor") is converted, through addition of potassium t-butylate into its respective potassium alcoholate. To the latter, sodium chloroacetate is added to give the respective cetirizine salt. From the so-obtained cetirizine salt, cetirizine or its hydrochloride and dihydrochloride can be obtained by addition of water and hydrochloric acid. However, in the aforementioned reaction, it is required to add the potassium t-butylate and the sodium chloroacetate alternately and in small portions, meticulously monitoring the reaction progress, which renders the process laborious if carried out on an industrial scale. Also, potassium t-butylate is a hazardous reagent such that its industrial use is undesirable, on top of being governed by specific safety restrictions. With this reaction, the achievable yield of cetirizine dihydrochloride (expressed in relation to the amount of ethanolic precursor employed) does not exceed, at any rate, 60%.

In view of these drawbacks, PL 163 415 proposed a synthetic approach according to which the ethanolic precursor was dissolved in toluene and added with a tenfold excess of solid sodium hydroxide. After three hours of boiling, 1.5 equivalents of chloroacetic acid in ethyl ether had to be added within one and a half hours to the boiling mixture. While the introduction of an etheric solution into a boiling toluene background poses again safety concerns arising from the appreciable difference between the boiling points of the two solvents, the yield of cetirizine dihydrochloride (always referred to the ethanolic precursor) does not exceed 60%, whereas 67% are within reach if unreacted precursor still present in the reaction mixture is recycled back. The persistence of residual amounts of ethanolic precursor (which can exceed, in the practice of PL 163 415, easily 10%) is however undesirable, as the ethanolic precursor is a problematic impurity, seen that in HPLC, the ethanolic precursor displays RT and rRT values practically identical to impurity "E" according to the Pharmacopeia. In other words, if it is not possible to run the reaction almost quantitatively, the required product quality becomes difficult to achieve, seen that the purification efforts required to impart the product pharmaceutically acceptable purity can easily skyrocket.

More recently, Applicant has proposed, in his Italian Patent Application MI2003A001558, an improved synthesis of cetirizine dihydrochloride employing the dihydrochloride of the ethanolic precursor as a starting substance and a high excess of inorganic base in toluene, to be reacted with a toluenic solution of chloroacetic acid. This process was able to reduce the residual unreacted ethanolic precursor in the reaction mixture to 5%-2% and to achieve, after adequate, proprietary extraction from the reaction mixture, a HPLC purity of the cetirizine dihydrochloride thereby obtained of at least 99.0%.

Yet, impurity B according to the Pharmacopeia remained around 0.3%. The achievable yield of cetirizine dihydrochloride (in relation to ethanolic precursor) with this reaction was of at least 70%.

Alternative more recent syntheses to produce cetirizine and its salts from the ethanolic precursor are described in the published applications WO 2004/103982, WO 2004/050647 and US 004/0266787:

WO 2004/103982 reacts the ethanolic precursor in a polar aprotic solvent (in particular DMF) with a haloacetate like sodium chloroacetate in the presence of potassium hydroxyde. The cetirizine base isolated from the reaction mixture is treated first with toluene and then treated with hexane in order to obtain a filtered solid showing a HPLC purity of 99 %, the yield reported (cetirizine base referred to ethanolic precursor), at this preliminary stage, being around 85%. Cetirizine dihydrochloride (the target compound of the present invention) is not prepared in WO 2004/103982, since the aim of this document is the proposal of the monohydrochloride as active principle. Moreover, WO 2004/103982 does not recite the levels of individual impurities still contained in the cetirizine base obtained according to its teaching.

WO 2004/050647 is concerned with the preparation of crystalline and amorphous forms of dextrorotatory and levorotatory cetirizine dihydrochloride salts. The proposed synthesis includes the reaction of the (levorotatory or dextrorotatory) ethanolic precursor in DMF with sodium monochloroacetate in the presence of an inorganic base (potassium hydroxide). According to this process, crude (levorotatory or dextrorotatory) cetirizine is obtained in a yield around 93,7-99,6%, and thereafter converted into its (levorotatory or dextrorotatory) dihydrochloride salt with a yield of the sole salification step of about 50%, thus sensibly reducing the overall yield in relation to the amount of ethanolic precursor employed, the achieved purity according to Pharmacopeia criteria not being specified.

US 2004/0266787 is likewise concerned with the attainment of amorphous forms of cetirizine and its salts. The chemical approach described therein is very similar to the one of WO 2004/050647.

WO 2004/050647 and US 2004/0266787 do not recite the levels of individual impurities contained in the cetirizine dihydrochloride obtained according to their teaching.

As it appears from what briefly outlined above, while considerable improvements have been achieved in recent times focussing on various goals, the industrial methods of synthesis discussed above for obtaining cetirizine dihydrochloride are not yet completely satisfactory in terms of both, yield and purity.
This is in particular because, in the meantime, the purity requirements in the European Pharmacopeia became more severe, spiking from a maximum of 0.2 % (HPLC) for any impurity peak (with a maximum of 0.3% for their sum) to 0.1% (HPLC), for the defined impurities A-F, as well as for further impurities, the maximum of their sum remaining still 0.3%.

As it appears from the above, it will be difficult to achieve these improved levels of purity after simple isolation of the cetirizine dihydrochloride from the reaction mixture, such that the newly required pharmaceutical purity is likely to require additional purification steps, what means downstream loss of yield and time.

It would hence be desirable to dispose of a process which yields pharmaceutically acceptable cetirizine dihydrochloride directly, and preferably in high yields after its isolation from the reaction mixture. Such process should also be highly reliable.

Reliability is an important issue because, not only the substantially quantitative completion of the reaction is difficult to achieve, but e.g. in the reaction system according to Italian Patent Application MI2003A001558, the conditions chosen are very sensible to the manifestation of cases in which the synthesis does not run satisfactorily.

Apart from being reliable, the new process should therefore also be able to keep down, already in the reaction mixture, impurity **B,** as much as possible, as this impurity is particularly difficult to eliminate downstream. On top of that, the new process should likewise be able to avoid "betainic impurities", not specifically considered by the Pharmacopeia, which may however result from the competing alkylation of at least one of the two tertiary nitrogen centers present in the ethanolic precursor of the cetirizine base's structure.

### Summary of the invention.

The above-mentioned problems have now been solved, and the above-identified targets have been achieved by the Applicant through the provision of the new, herein described process for the synthesis of cetirizine dihydrochloride salt from the ethanolic precursor of cetirizine, according to which:
(a) a solution of {2-[4-(α-phenyl-p-chlorobenzyl)piperazin-1-yl]}ethanol in 1-7 volumes, referred to the weight of {2-[4-(α-phenyl-p-chlorobenzyl)piperazin-1-yl]}ethanol, of an organic solvent having a boiling point higher than 90°C and being chosen from the group consisting of aliphatic, cycloalifatic or aromatic solvents is provided, whereafter
(b) per equivalent of {2-[4-(α-phenyl-p-chlorobenzyl)piperazin-1-yl]}ethanol employed, 1-2 equivalents of metal haloacetate or of haloacetic acid, as well as 3-7 equivalents of NaOH are added to the solution as per (a), providing a reaction mixture, whereafter 0,05-0,3 volumes, referred to the weight of {2-[4-(α-phenyl-p-chlorobenzyl)piperazin-1-yl]}ethanol employed, of water and 0,1-1,2 volumes, referred to the weight of {2-[4-(α-phenyl-p-chlorobenzyl)piperazin-1-yl]}ethanol employed, of a polar aprotic, water miscible solvent are added, keeping the internal temperature of the reaction mixture below 60°C, whereafter
(c) the cetirizine base formed within the reaction mixture is converted into its hydrochloride salt and isolated.

### Detailed description of the invention.

As pointed out above, with the switch from European Pharmacopeia 4 to Pharmacopeia 5.0, a synthetic approach converting the ethanolic precursor of cetirizine into cetirizine dihydrochloride salt of pharmaceutical grade without the need of downstream purification, was no longer available.

On top of that, the yields of known methods of synthesis of cetirizine and its (di)hydrochloride salt from its ethanolic precursor still needed improvement. A serious problem at least with some of the known reactions of this type was further that they were not reliable in the sense that ignition of the reaction and/or completion did not always take place within reasonable time.
For example, Applicant has found that when the ethanolic precursor of cetirizine is reacted, as foreseen by WO 2004/103982 with sodium chloro acetate in about three volumes (referred to the amount of ethanolic precursor employed) of aprotic polar solvent (used solvent: DMSO), the reaction is sometimes successfully completed (in terms of the disappearance of the initially employed ethanolic precursor) after 24 hours, sometimes only one half of the starting product is consumed. On top of not being clear, on which feature the said unsatisfactory reliability depends, in the said reaction appreciable amounts of betainic impurity (decidedly more than 1 %) forms which cannot be eliminated in course of the upstream work -such that the finally obtained cetirizine dihydrochloride is in the need of further purification steps.
Other tests performed with different amounts of an aprotic dipolar solvent (and specifically with DMSO) have led sometimes to a partial reaction or, when a complete reaction is obtained, have led to the formation of relevant amounts of the above mentioned unacceptable betainic impurity.

Applicant has now found in unexpected manner that, with his herein described process, the conversion of the ethanolic precursor of cetirizine into the desired product proceeds reliably and in reproducible manner within reaction times which do not exceed 24 hours, preferably 20 hours. Applicant has also found that within the aforementioned time span, it is possible to reliably arrive at almost quantitative consumption of the initially employed ethanolic precursor of cetirizine. Levels of unreacted ethanolic precursor of 3-0 % in the reaction mixture are deemed acceptable as these levels (i) not substantially hamper the achievement of high overall yields of the desired product (80% and more of cetirizine dihydrochloride, related to the initially employed ethanolic precursor), and (ii) are lost during the downstream work-up such as not to "inflate" the HPLC peak of impurity E beyond the threshold allowed by the Pharmacopeia. Such levels of residual unreacted ethanolic precursor at the end of the reaction does thus not make up a major challenge for the purity of the product.
More importantly, Applicant has also found that with his herein described process, it is possible to reduce impurity B according to the Pharmacopeia, as well as a betainic impurity (resulting from the competing alkylation of at least one of the two tertiary nitrogen centers present in the ethanolic precursor of the cetirizine base's structure) to very low amounts (less than 0,2%) in the spent reaction mixture. Since these impurities are very hard to eliminate in the downstream work-up, their almost complete suppression achieved by the present invention at the end of step (b) is of paramount importance to arrive, after the work-up, directly to the desired cetirizine dihydrochloride of pharmaceutical grade. To bring about the above achievements, Applicant proposes hence a new process for the synthesis of cetirizine dihydrochloride salt from the ethanolic precursor of cetirizine, according to which:
(a) a solution of {2-[4-(α-phenyl-p-chlorobenzyl)piperazin-1-yl]}ethanol in 1-7 volumes, referred to the weight of {2-[4-(α-phenyl-p-chlorobenzyl)piperazin-1-yl]}ethanol, of an organic solvent having a boiling point higher than 90°C and being chosen from the group consisting of aliphatic, cycloalifatic or aromatic solvents is provided, whereafter
(b) per equivalent of {2-[4-(α-phenyl-p-chlorobenzyl)piperazin-1-yl]}ethanol employed, 1-2 equivalents of metal haloacetate or of haloacetic acid, as well as 3-7 equivalents of an alkaly metal hydroxyde base are added to the solution as per (a), providing a reaction mixture, where 0,05-0,3 volumes, referred to the weight of {2-[4-(α-phenyl-p-chlorobenzyl)piperazin-1-yl]}ethanol employed, of water and 0,1-1,2 volumes, referred to the weight of {2-[4-(α-phenyl-p-chlorobenzyl)piperazin-1-yl]}ethanol employed, of a polar aprotic, water miscible solvent are added, keeping the internal temperature of the reaction mixture below 60°C, whereafter
(c) the cetirizine base formed within the reaction mixture is converted into its dihydrochloride salt and isolated.
   In the above process, it is critical to employ a ternary system of solvents as specified and to keep their above-identified proportions such as to meet the above-stated goals. For example, if the amount of water added to the system is too high, the reaction will not "run".

Further, embodiments of the herein described process are preferred wherein, 1-7 preferably 2-4 volumes of organic solvent are employed.

Further, embodiments of the herein described process are preferred wherein, 1-2 preferably 1,3-1,7 equivalents of metal haloacetate or haloacetic acid are employed.

Further, embodiments of the herein described process are preferred wherein 3-7, preferably 3,5-6 equivalents of an alkaly metal hydroxyde base are employed.

Further, embodiments of the herein described process are preferred wherein 0,05-0,3, preferably 0,10-0,25 volumes of water are employed.

Further, embodiments of the herein described process are preferred wherein 0,1-1,2 preferably 0,2-1 volumes of a polar aprotic, water miscible solvent are employed.

Further, embodiments of the herein described process are preferred wherein the organic solvent having a boiling point higher than 90° C is chosen from alkyl benzenes and halogen benzenes.

Further, embodiments of the herein described process are preferred wherein the organic solvent having a boiling point higher than 90°C is chosen from the group consisting of toluene, xylene, ethylbenzene and chlorobenzene.

A particularly preferred embodiment of the herein described process employs toluene as organic solvent having a boiling point higher than 90° C.

Further, embodiments of the herein described process are preferred wherein the metal haloacetate is chosen from alkali and earth alkali acetate.

Further, embodiments of the herein described process are preferred wherein the halogen in the halo acetate or haloacetic acid is chosen from fluorine, chlorine, bromine and iodine.

A particularly preferred embodiment of the herein described process employs sodium chloro acetate.

Further, embodiments of the herein described process are preferred wherein the polar aprotic solvent is chosen from the group consisting of DMSO, DMF and DMA, DMSO being particularly preferred.

### Examples.

### Example 1 (invention).

### Synthesis of cetirizine dihydrochloride (M.W. 461,80):

### (a) Formation of the base of the ethanolic precursor.

Charge into a first reaction flask:

| 2-[4-(4-chlorophenyl)-phenylmethyl]-1-piperazinyl)ethanol dihydrochloride | | |
|---|---|---|
| (MW: 403,79; moles: 0,30 ) | g | 121 |
| deionized water | cc | 250 |
| toluene | cc | 300 |
| sodium hydroxide microprills | g | 24,8 |
| ( PM: 40 moles : 0,62 ) | | |

heat to 55 - 60 °C and let the phases separate, keep the organic phase for the next reaction step.

Obtained : g 356,2 of toluenic solution of:
2-[4-(4-chlorophenyl)-phenylmethyl]-1-piperazinyl)ethanol base.

### (b) Reaction.

Charge a second flask with the following:

| | | | |
|---|---|---|---|
| - | sodium chloroacetate | g | 55,87 |
| | (MW: 116,48 moles: 0,48 ) | | |
| - | sodium hydroxide microprills (MW: 40 moles : 1,5 ) | g | 59,96 |

- and the toluenic solution of 2-[4-(4-chlorophenyl)-phenylmethyl]-1-piperazinyl)ethanol base obtained before.

| | | | | |
|---|---|---|---|---|
| - | drip first, letting the internal temperature raise to 30 - 35 °C: | | | |
| - | deionized water | | cc | 20 |
| and thereafter, always keeping the internal temperature at 30 - 35 °C: | | | | |
| - | DMSO | | cc | |
| | | 31,25 | | |

- stir for 14 - 18 hours, always keeping the internal temperature at 30 - 35 °C.

| *end of reaction control (HPLC):* | | | |
|---|---|---|---|
| *Cetirizine* | *: 99, 62 %;* | *unreacted ethanolic precursor* | *: 0,05 %;* |
| *betainic impurity* | : *0, 08 %;* | *impurity B: 0 %* | |
| *impurity A* | : *0,17* % | | |
| *(impurity A is an impurity which is easily eliminated in the further course of work-up).* | | | |

### (c) Work-up.

Add, to the reaction mixture resulting from step (b) above:

| | | |
|---|---|---|
| - | deionized water | cc |
| | 160 | |

and let separate, without cooling, the salt-charged aqueous phase, discarding the same.

Add then to the rich toluenic phase:

| | | | |
|---|---|---|---|
| - | water | | cc |
| | | 160 | |

and bring the pH value to 6,8 - 7,0 with

| | | |
|---|---|---|
| | - hydrochloric acid 36 % | q.b. |

and then separate the phases without cooling.

| | | | |
|---|---|---|---|
| - | Wash the rich aqueous phase with: | | |
| - | Toluene, | cc | 100 x 2 |
| | times | | |

let the phases separate, cool the rich aqueous phase to RT, and add thereto:

| | | | |
|---|---|---|---|
| - | methylene chloride cc | | |
| | | 600 | |

then bring the pH value to 4 - 4,3 with:
- hydrochloric acid 36 %
q.b.
and separate the rich chloromethylenic phase. Wash the rich chloromethylenic phase with:

| | | | |
|---|---|---|---|
| - | Water, | cc | 100 x 2 |
| | times | | |

separating and discarding the aqueous phase. Add:

| | | | |
|---|---|---|---|
| - | hydrochloric acid 36 %, | | cc |
| | | 35 | |

then distill off the methylene chloride first under atmospheric, then under reduced pressure.

Add to the stripped residue thereby obtained:

| | | | |
|---|---|---|---|
| | - acetone | | cc |
| | | 900 | |

- then, add additional:

| | | | |
|---|---|---|---|
| - | hydrochloric acid 36 % | | cc |
| | | 9 | |

- stir over night at RT and then filter on Büchner funnel washing with:

| | | | |
|---|---|---|---|
| | - acetone | | cc |
| | | 200 | |

and dry the wet product at 60 °C in the vacuum.

| | | | | |
|---|---|---|---|---|
| Obtained: | wet | g | 215,6 | of Cetirizine dihydrochloride |
| | dry | g | 118,8 | white colour |

- Yield = 85,8 % of theory: (referred to 2-[4-(4-chlorophenyl)-phenylmethyl]-1-piperazinyl)ethanol **dihydrochloride**)
- *Yield = 87,4 % of theory*: *(referred to 2-[4-(4-chlorophenyl)-phenylmethyl]-1-piperazinyl)ethanol **base** really contained within the toluenic solution according to step **(a)***

**HPLC** purity: 99,78 %; max. value of a single impurity: 0,07 %

### Examples 2-23 (invention and comparative)

The following table sets out the results of further tests in which the Reaction as per step (b) was carried out in different conditions as specified.
The examples 6, 7, 8, 18, 19 are illustrative of the invention while the examples 2, 3, 4, 5, 9, 10, 11, 12, 13, 14, 15, 16, 17, 20, 21, 22, 23 are comparative examples which ) demonstrate the criticity of the claimed reaction conditions.

| **Example Number** | **Toluene vol.** | **DMSO vol.** | **Water vol.** | **Base employed** | **Moles of base** | **Reagent employed** | **Moles of reagent** | **Reaction temperature** | **Unreacted ethanolic precursor base at the end of step (b)** | **Reaction time in hours** | **impurity B according to Pharmacopeia** | **Betainic impurity** |
|---|---|---|---|---|---|---|---|---|---|---|---|---|
| 2 (comparative) | 0 | 1 | 0 | NaOH | 3 | Na chloroacetate | 1,54 | RT | 68% | 20 | 0,03% | 0,54% |
| 3 (comparative) | 0 | 1 | " | " | 5,1 | " | 2 | " | 67% | 20 | 0,03% | 0,49% |
| 4 (comparative) | 2,3 | 1 | " | " | 3 | " | 1,54 | " | 99% | 3 | 0,00% | 0,03% |
| 5 (comparative) | 0 | 1,2 | " | " | 8,2 | " | " | 40°C | 36% | 20 | 0,11% | 3,39% |
| 6 (invention) | 3 | 0,5 | 0,157 | " | 5,65 | chloroacetic acid | " | 30-35°C | 2,20% | 20 | 0,01% | 0,08% |
| 7 (invention) | 3 | 0,5 | 0,1179 | " | " | " | " | " | 3,11% | 20 | 0% | 0,07% |
| 8 (invention) | 3 | 0,5 | 0,0786 | " | 5,65 | " | " | " | 3,50% | 20 | 0,01% | 0,08% |
| 9 (comparative) | 0 | 3 | 1 | " | 4,1 | Na chloroacetate | " | " | 50% | 20 | 0,08% | 0,24% |
| 10 (comparative) | 0 | 0 | 4 | " | " | " | " | " | 100% | 20 | 0,02% | 0,03% |
| 11 (comparative) | 2 | 0 | 2 | " | " | " | " | " | 100% | 20 | 0% | 0% |
| 12 (comparative) | 0 | 1,2 | 0 | KOH | 3,4 | Na chloroacetate | " | " | 0,25% | 26 | 0% | 0,35% |
| 13 (comparative) | 0 | 3,6 | 0,322 | NaOH | 4,1 | " | " | " | 16% | 26 | 0,03% | 2,33% |
| 14 (comparative) | 0 | 0 | 4 | " | " | " | " | 80-90°C | 99,50% | 5 | 0% | 0,24% |
| 15 (comparative) | 2 | 0 | 2 | " | " | " | " | " | 98,50% | 5 | 0% | 0,21% |
| 16 (comparative) | 2 | 0 | 0 | " | 3 | " | " | " | 72% | 5 | 0,07% | 0,22% |
| 17 (comparative) | 0 | 3 | 1 | KOH | 4,1 | " | " | 30-35°C | 59% | 18 | 0,17% | 0,03% |
| 18 (invention) | 6 | 0,5 | 0,16 | NaOH | " | " | " | " | 0,93% | 20 | 0,01% | 0,09% |
| 19 (invention) | 1,5 | 0,5 | 0,16 | " | " | " | " | " | 0,27% | 20 | 0,03% | 0,07% |
| 20 (comparative) | 6 | 0,5 | 1,6 | " | " | " | " | " | 100% | 20 | 0% | 0,01% |
| 21 (comparative) | 0 | 1,8 | 0 | KOH | 3,4 | " | " | " | 26,70% | 20 | 0,14% | 2,83% |
| 22 (comparative) | 0 | 1,8 | 0 | " | " | " | " | " | 26% | 24 | 0,18% | 4,80% |
| 23 (comparative) | 0 | 3,25 | 0 | " | 2,43 | " | " | " | 52% | 24 | 0,14% | 5,90% |

| | | | | | | | | | | | | |
|---|---|---|---|---|---|---|---|---|---|---|---|---|
| **NOTE 1:** The volumes of toluene, DMSO and water are each referred to one part by weight of ethanolic precursor base (as obtained after step (a)) **NOTE 2:** The moles of base and of reagent are referred to the ethanolic precursor base (as obtained after step (a)) **NOTE 3:** The two impurities which unlike others are difficult to eliminate from the reaction mixture in the downstream treatment are impurity B and the betainic impurity. | | | | | | | | | | | | |

## Claims

1. Process for the synthesis of cetirizine dihydrochloride, wherein
(a) a solution of {2-[4-(α-phenyl-p-chlorobenzyl)piperazin-1-yl]}ethanol in 1-7 volumes, referred to the weight of {2-[4-(α-phenyl-p-chlorobenzyl)piperazin-1-yl]}ethanol, of an organic solvent having a boiling point higher than 90°C and being chosen from the group consisting of aliphatic, cyclo alifatic or aromatic solvents is provided, whereafter
(b) per equivalent of {2-[4-(α-phenyl-p-chlorobenzyl)piperazin-1-yl]}ethanol employed, 1-2 equivalents of metal haloacetate or of haloacetic acid, as well as 3-7 equivalents of an alkali metal hydroxyde base added to the solution as per (a), providing a reaction mixture, where 0,05-0,3 volumes, referred to the weight of {2-[4-(α-phenyl-p-chlorobenzyl)piperazin-1-yl]}ethanol employed, of water and 0,1-1,2 volumes, referred to the weight of {2-[4-(α-phenyl-p-chlorobenzyl)piperazin-1-yl]}ethanol employed, of a polar aprotic, water miscible solvent are added, keeping the internal temperature of the reaction mixture below 60°C, whereafter
(c) the cetirizine base formed within the reaction mixture is converted into its dihydrochloride salt and isolated as such.

2. Process according to claim 1 wherein 2-4 volumes of organic solvent are employed.

3. Process according to claim 1 wherein 1,3-1,7 equivalents of metal haloacetate or haloacetic acid are employed.

4. Process according to claim 1 wherein 3,5-6 equivalents of an alkali metal hydroxide base are employed.

5. Process according to claim 1 wherein 0,10-0,25 volumes of water are employed.

6. Process according to claim 1 wherein 0,2-1 volumes of a polar aprotic, water miscible solvent are employed.

7. Process according to claim 1 wherein the organic solvent having a boiling point higher than 90° C is chosen from alkyl benzenes and halogen benzenes.

8. Process according to claim 7, wherein the organic solvent is chosen from the group consisting of toluene, xylene, ethylbenzene and chlorobenzene.

9. Process according to claim 7, wherein toluene is employed as the organic solvent.

10. Process according to claim 1 wherein the metal haloacetate is chosen from alkali and earth alkali haloacetates.

11. Process according to claims 10 wherein the halogen is chosen from fluorine, chlorine, bromine and iodine.

12. Process according to claim 1 wherein the alkali metal hydroxide base is NaOH.

13. Process according to claim 1 wherein the polar aprotic solvent is chosen from the group consisting of DMSO, DMF and DMA.

14. Process according to claim 13 wherein the polar, aprotic solvent is DMSO.
